# EUROPEAN PATENT SPECIFICATION

(11) **EP 1 861 141 B1**
(45) Date of publication and mention of the grant of the patent: **18.12.2013**
(21) Application number: 06706054.1
(22) Date of filing: 14.02.2006
(51) Int. Cl.: A61M 5/315

(54) **A DOSE SETTING ELEMENT FOR AN INJECTION DEVICE AND HAVING A DOSE SETTING LIMITING MECHANISM**
DOSISEINSTELLELEMENT FÜR EINE INJEKTIONSVORRICHTUNG MIT DIE DOSISEINSTELLUNG BEGRENZENDEM MECHANISMUS
ELEMENT D'AJUSTEMENT DE DOSAGE POUR UN DISPOSITIF D'INJECTION ET POURVU D'UN MECANISME DE LIMITATION D'AJUSTEMENT DE DOSAGE

(30) Priority: 17.02.2005 DK 200500243
(43) Date of publication of application: 05.12.2007
(73) Proprietor: Novo Nordisk A/S, 2880 Bagsvaerd (DK)
(72) Inventor: RADMER, Bo, 3400 Hillerød (DK); MØLLER, Claus Schmidt, 3480 Fredensborg (DK)
(86) International application number: PCT/DK2006/000083
(87) International publication number: WO 2006/086983

(56) References cited:
- WO-A-99/38554
- WO-A-2004/002557
- WO-A-2004/078241
- US-A1- 2004 210 199

## Description

### FIELD OF THE INVENTION

The present invention relates to a dose setting element for an injection device, e.g. an injection device being suitable for self injection, such as a device for injecting insulin. The dose setting element comprises a mechanism for limiting the setting of a dose beyond a predefined total amount. The present invention further relates to an injection device with such a dose setting element.

### BACKGROUND OF THE INVENTION

In injection devices with a cartridge inserted therein, the cartridge containing enough liquid for multiple injections, it is sometimes desirable to be able to prevent that a dose is set which exceeds the amount of liquid left in the cartridge. If a dose is set which exceeds the amount of liquid left in the cartridge the user will believe that a certain dose will be injected, while in fact a lower dose will be injected. This is highly undesirable and may be dangerous or even lethal.

Previously it has been attempted to solve this problem by providing the injection device with a dose limiting mechanism connected to the dose setting element in such a way that the total amount of doses set are counted, and when the total amount equals the amount contained in the cartridge the limiting mechanism abuts a stop member, thereby preventing further setting of a dose.

An example of such a device is described in WO 2004/078226 disclosing a drive mechanism for use in drug delivery devices. The drive mechanism comprises a housing, a dose dial sleeve and a two-part piston rod. In one embodiment a drive sleeve descends on an inner part of the piston rod when a dose is set. The distance travelled is equal to the distance required to displace the cartridge piston to expel the selected dose. When a subsequent dose is selected the drive sleeve advances further along the inner part of the piston rod. The position of the drive sleeve corresponds to the amount of medicinal product left in the cartridge. When the drive sleeve reaches the end of a threaded portion of the inner part of the piston rod and can rotate no further, this corresponds to no medicinal product remaining in the cartridge.

Another example of such a device is described in US 6,582,404 disclosing a limiting mechanism which prevents the setting of a dose, which exceeds the amount of liquid left in a cartridge of an injection device. The injection device is the type where a dose is set by rotating a dose setting member relative to a driver and away from a fixed stop in the injection device. The dose setting member interfaces the driver such that the dose setting member can be rotated in one direction without rotating the driver. The dose is injected by rotating back the dose setting member which during backward rotation carries the driver with it. Rotating the driver causes the piston rod to move forward inside the cartridge and expel some of the liquid contained in the cartridge. The driver is provided with a track having a length which is related to the total amount of liquid in the cartridge and which track is engaged by a track follower coupled to the dose setting member to follow rotation of this dose setting member. Each time a dose is set and injected, the track follower moves further into the track. When the track follower reaches the end of the track the dose setting member can not be rotated further, and a dose larger than the remaining liquid in the cartridge cannot be set.

Yet another example of such a device is described in EP 0 554 996 disclosing an injection device for injecting fluids such as insulin within body tissue. The injection device comprises a dose setting mechanism including a units counter ring and a tens counter ring. A transmission key is provided for selectively coupling the units and the tens counter rings so that they rotate together only during selected portions of the dose setting procedure. The set dose is shown by means of the units counter ring and the tens counter ring. The injection device further comprises a dose limiting mechanism which limits the travel of a lead screw to the safe travel of the piston in the cartridge. If the lead screw reaches the end of its travel, projections inside the plunger reach the end of some grooves in the lead screw and prevent it from further movement. This mechanism is separate from the counter rings.

A dose setting element according to the preamble of claim 1 is known from document WO 2004/002557 A.

It is a disadvantage of the limiting mechanisms disclosed in WO 2004/078226, US 6,582,404 and EP 0 554 996 that the mechanisms take up a lot of space in the respective injection devices because at least one part of the mechanisms needs to travel a distance corresponding to the total set dose.

### SUMMARY OF THE INVENTION

It is, thus, an object of the present invention to provide a dose setting element having a mechanism for limiting the setting of a dose beyond an amount left in a cartridge, where the limiting mechanism is less space consuming than prior art limiting mechanisms.

It is a further object of the present invention to provide a compact dose setting element having a mechanism for limiting the setting of a dose beyond an amount left in a cartridge.

It is an even further object of the present invention to provide an injection device comprising a mechanism for limiting the setting of a dose beyond an amount left in a cartridge, the injection device being compact as compared to prior art injection devices.

According to a first aspect of the present invention the above and other objects are fulfilled by providing a dose setting element for an injection device, the dose setting element being operable to set a desired dose to be injected by the injection device, the dose setting element comprising:
- a first part being adapted to be rotated during the setting of a dose,
- a second part being adapted to limit setting of a dose beyond a predefined total amount, the first part and the second part being adapted to interengage in such a way that each time the first part has been rotated through a first specific angle the second part is caused to be rotated through a second specific angle, the second specific angle being smaller than the first specific angle, and
- a stop member being adapted to abut the second part when the second part has been rotated through an angle being equal to the second specific angle a predefined number of times in total, thereby limiting the second part from further rotational movement and thereby preventing further dose setting.

The first part is adapted to be rotated during the setting of a dose. Preferably, the injection device is of a kind where the dose is set by rotating a dose setting member, e.g. a dose knob, relatively to a housing. In this case the first part is preferably rotated along with the dose setting member during the setting of a dose. Though a dose setting member as described above will normally perform a translational movement along a longitudinal axis of the injection device during dose setting in addition to the rotational movement, the first part will preferably not perform such a translational movement. It therefore does not take up much space in the injection device.

The second part is adapted to limit setting of a dose beyond a predefined total amount. This should be understood in the following way. The injection device is of the kind where the cartridge contains sufficient liquid for multiple injections. The second part ensures that when a dose is to be set this dose added to all the previous doses which have been set since the present cartridge was inserted in the injection device does not exceed the predefined total amount. The predefined total amount may correspond to the amount contained in a full cartridge. In this case the second part prevents setting of a dose beyond the amount left in the cartridge.

Each time the first part is rotated through a first specific angle the second part is caused to be rotated through a second specific angle. The second specific angle is smaller than the first specific angle. Thereby the first part can be rotated through a relatively large angle while the second part is only rotated through a relatively small angle. The angular position of the first part and the angular position of the second part in combination indicate the total dose set since the present cartridge was inserted in the injection device, i.e. they 'keep track' of how much liquid remains in the cartridge. It may, however, not be possible for a user to follow the remaining amount of liquid during the entire process. The important thing is that the first and the second part in combination 'know' how much liquid is remaining, and that they accordingly prevent setting of a dose beyond the predefined total amount. Thus, the first part may be rotated through several revolutions in total while the second part is rotated through a much smaller angle, e.g. less than one revolution in total. Thereby neither the first part nor the second part needs to be moved accumulated in an axial direction, and the total limiting mechanism (including the first part and the second part) therefore takes up very little space in the injection device as compared to prior art limiting mechanisms. Thereby the dose setting element, and in turn the injection device, can be made in a compact manner. Typically, the limiting mechanism functions in such a way that each time the first part has been rotated through one revolution the second part is caused to be rotated through a smaller angle (e.g. by rotating along with the first part for a while). When the second part has been rotated in total through one revolution it will abut the stop member. This abutment prevents the second part from rotating, which in turn prevents the first part from rotating, thereby preventing further setting of a dose. However, the second part may alternatively be rotated continuously, but at a slower pace than the first part, e.g. by means of a gearing arrangement. It should also be understood that the specific angles mentioned above are merely examples, and that other angles could be envisaged. The important feature is that the second part is rotated through a smaller angle than the first part, and that this allows the limiting mechanism to be small.

It is, thus, an advantage of the invention that the limiting mechanism comprises the first part and the second part being adapted to interengage as described, because this allows the limiting mechanism to be relatively small, thereby allowing for a relatively compact dose setting member, and thereby a relatively compact injection device.

It should be understood that the dose setting element may further comprise a third part being adapted to engage with the first part as well as the second part in such a way that the engagement between the first part and the second part is established via the third part. Thus, the third part functions as an 'intermediate' part. In this case the limiting mechanism will function in the following manner. Each time the first part has been rotated through a first specific angle the third part is caused to be rotated through a third specific angle, and each time this has occurred a specific number of times, the second part is caused to be rotated through a second specific angle. The third specific angle should in this case be smaller than the first specific angle, and the second specific angle should be smaller than the third specific angle times the specific number. Whereas the limiting mechanism described above functions similarly to a counter having a 'unit wheel' (the first part) and a 'tens wheel' (the second part) this limiting mechanism functions similarly to a counter having a 'unit wheel' (the first part), a 'tens wheel' (the third part) and a 'hundreds wheel' (the second part). It should be understood that the limiting mechanism could comprise any suitable number of additional 'Intermediate' parts similar to the third part described above.

The predefined number of times may correspond to a predefined amount of liquid being left in the cartridge. The predefined amount of liquid may, e.g., be chosen in such a way that it is ensured that it is possible to set a full dose when the second part and the stop member do not abut, or in such a way that it is no longer possible to mix the contents of the cartridge properly when the second part and the stop member abut, or according to any other suitable criteria. The predefined amount of liquid may, alternatively, be chosen as an amount of liquid which may be left in a shoulder of the cartridge, and which it is therefore, in practice, not possible to retract from the cartridge.

Alternatively or additionally, the predefined number of times may correspond to the amount of liquid present in a cartridge. In this case the cartridge will be at least substantially empty when the stop member and the second part abut. This should be interpreted to include the situation described above where a small, non-retractable, amount of liquid is left in the cartridge, e.g. in a shoulder of the cartridge.

The dose setting element may further comprise means for moving the first part and/or the second part into a position where the first part and the second part interengage, thereby causing the second part to be rotated along with the first part through an angle being equal to the second specific angle. It should be understood that this could also be interpreted in such a way that a part of the first and/or the second part (e.g. a flexible arm positioned on or being connected to the first or the second part) is moved into engagement with the other part when the first and/or the second part is/are in (a) specified angular position(s). Alternatively, the whole of the first and/or second part may be moved in order to cause the engagement between the parts.

The moving means may comprise one or more protruding parts positioned on the first part and adapted to cooperate with one or more corresponding protruding parts positioned on the second part, the protruding parts in cooperation causing the movement of the first and/or the second part.

In one embodiment the first part and the second part are adapted to interengage by means of mating teeth positioned on the first part and the second part, respectively. When the first and/or second part is/are moved in such a way that the parts approach each other, the teeth will engage, and the second part will be rotated along with the first part until the first and the second parts are no longer engaged.

Alternatively, the first part and the second part may be adapted to interengage by means of one or more arms positioned on the first or second part and being adapted to engage with one or more corresponding members on the other part. The corresponding member(s) may be a set of teeth, one or more arms, one or more grooves, etc.

The one or more arms may form part of the first part. Alternatively it/they may form part of the second part, or it/they may form a separate member connected to the first or the second part.

Preferably, the first part and the second part are adapted to rotate about a common rotational axis. This common rotational axis preferably coincides with a longitudinal axis of the injection device.

In one embodiment the first part may be or form part of a dose setting member, e.g. a dose knob. In this embodiment the second part is rotated through the second specific angle each time the dose setting member is rotated through the first specific angle.

According to a second aspect of the present invention the above and other objects are fulfilled by providing an injection device comprising:
- a housing,
- a dose setting element according to the first aspect of the present invention, and
- a piston rod being adapted to cooperate with a piston so as to cause a set dose to be injected by the injection device.

The injection device preferably has an elongated shape. Thus, it may be a pen-like device.

### BRIEF DESCRIPTION OF THE DRAWINGS

The invention will now be further described with reference to the accompanying drawings in which:
Fig. 1 shows a dose limiting mechanism according to a first embodiment of the invention with a first and a second part,
Fig. 2 shows the first part of the dose limiting mechanism of Fig. 1,
Fig. 3 shows the second part of the dose limiting mechanism of Fig. 1,
Fig. 4 shows the dose limiting mechanism of Fig. 1 inserted into an injection device, the first and the second parts being in a non-engaged position,
Fig. 5 shows the dose limiting mechanism of Fig. 1 inserted into an injection device, the first and the second parts being in an engaged position,
Figs. 6 and 7 show various parts of an injection device having a dose limiting mechanism according to a second embodiment of the invention.

### DETAILED DESCRIPTION OF THE DRAWINGS

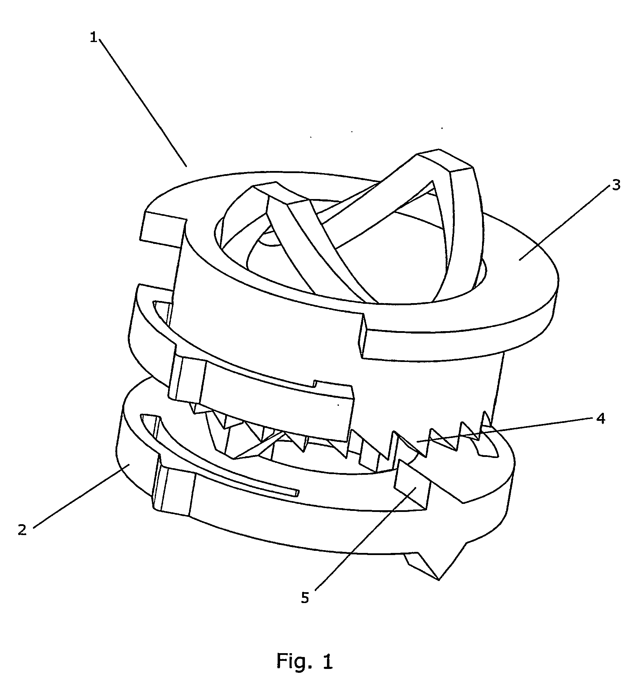

Fig. 1 shows a dose limiting mechanism 1 having a first part 2 and a second part 3. The first part 2 is adapted to be rotated along with a dose setting member (not shown) during setting of a dose. The second part 3 is provided with a set of teeth 4 which are adapted to engage with mating teeth 5 positioned on the first part 2. Only one mating tooth 5 is visible in Fig. 1. In Fig. 1 the first part 2 and the second part 3 are positioned relatively in such a way that the set of teeth 4 and the mating teeth 5 are not engaging. When the first part 2 is rotated the second part 3 will therefore not rotate along with the first part 2. However, the first part 2 may be moved in a direction towards the second part 3 until the set of teeth 4 and the mating teeth 5 engage. This movement will be explained in further detail below. When the first part 2 is in this position the second part 3 will be rotated along with the first part 2 until the first part 2 is again moved in a direction away from the second part 3, thereby causing the set of teeth 4 and the mating teeth 5 to be moved out of engagement. As a result, the second part 3 has been rotated through an angle which is equal to the angle travelled by the first part 2 during the engagement of the set of teeth 4 and the mating teeth 5. But because the teeth 4, 5 are moved in and out of engagement, the angle travelled by the second part 3 is smaller than the angle travelled by the first part 2.

When the second part 3 has been rotated as described above a certain number of times, i.e. when a certain total angle has been travelled by the second part 3, the second part 3 will abut a stop member (not shown in Fig. 1). This abutment prevents the second part 3 from rotating further, thereby also preventing the first part 2 from rotating further when the first part 2 is in the position where the set of teeth 4 and the mating teeth 5 engage. Since the first part 2 is rotated during setting of a dose, this will in turn prevent the setting of a further dose. If the maximum angle travelled by the second part 3 corresponds to the amount of liquid in a cartridge, the dose limiting mechanism 1 thereby prevents the setting of a dose beyond the amount of liquid in the cartridge. Furthermore, because the second part 3 is only rotated through a small angle each time the first part 2 is rotated through a larger angle, the total angle travelled by the second part 3 is relatively small, typically less than or equal to 360°. There is therefore no need for either the first part 2 or the second part 3 to be moved in an axial direction, and the complete dose limiting mechanism therefore takes up very little space, and it is therefore possible to provide a very compact dose setting mechanism.

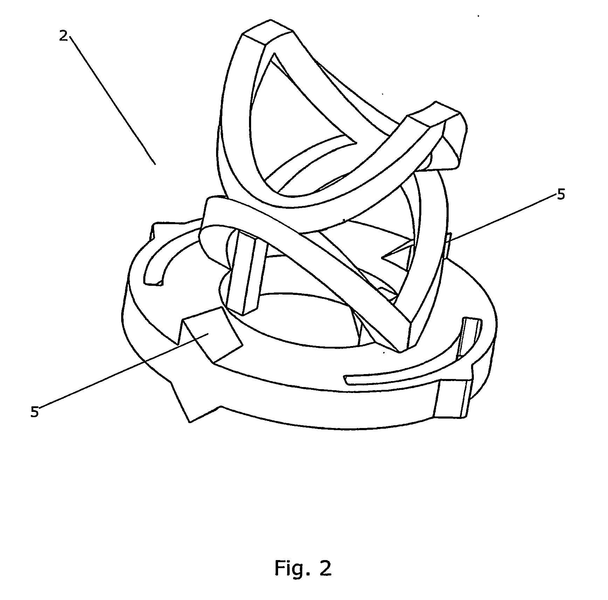

Fig. 2 shows the first part 2 of the dose limiting mechanism 1 of Fig. 1. The first part 2 is provided with two mating teeth 5 positioned at opposing angular positions. This positioning ensures that when the mating teeth 5 engage the set of teeth 4 on the second part 3 (see Fig. 1) the second part 3 will be rotated along with the first part 2 in a very stable manner.

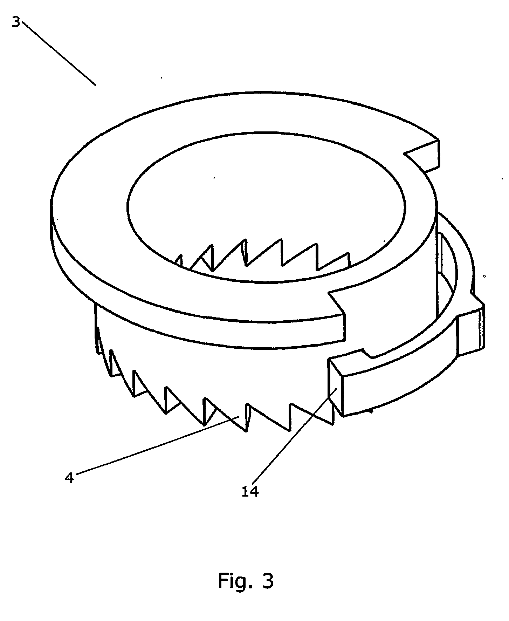

Fig. 3 shows the second part 3 of the dose limiting mechanism 1 of Fig. 1. As can be seen the set of teeth 4 extends the entire lower periphery of the second part 3. This ensures that regardless of the angular position of the second part 3 the set of teeth 4 and the mating teeth 5 of the first part 2 will engage when the first part 2 is moved into the engaging position. Furthermore, the second part 3 is provided with a stopping surface 14. The stopping surface 14 is adapted to abut a corresponding surface positioned inside a housing of the injection device when the stopping surface 14 is in a particular angular position. When the stopping surface 14 abuts the corresponding surface, the second part 3 is prevented from performing further rotational movement.

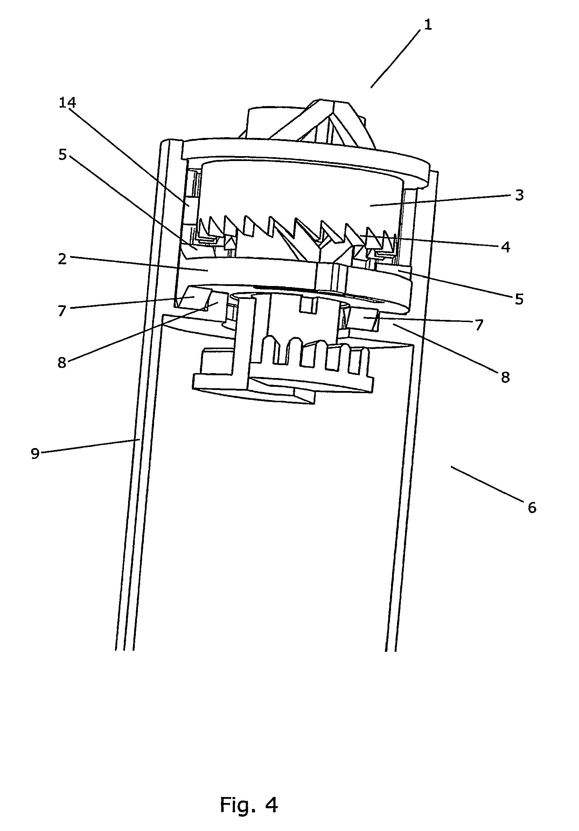

Fig. 4 shows the dose limiting mechanism 1 of Fig. 1 inserted into an injection device 6. The first part 2 is in a position where the set of teeth 4 and the mating teeth 5 are not engaged. The first part 2 is provided with two tapered members 7 being adapted to engage with corresponding tapered members 8 on a housing 9 of the injection device 6. The two tapered members 7 on the first part 2 as well as the two tapered members 8 on the housing 9 are positioned at different radial positions. Thereby the tapered members 7, 8 will only engage when the angular positions of corresponding tapered members 7, 8 coincide. On the other hand, when the first member 2 is in an opposing angular position the tapered members 7, 8 can freely pass each other as illustrated in Fig. 4, i.e. the tapered members 7, 8 will not engage. Thus, in the injection device 6 shown in Fig. 4, the tapered members 7, 8 will engage once every time the first part 2 has rotated 360°. This will be described further below.

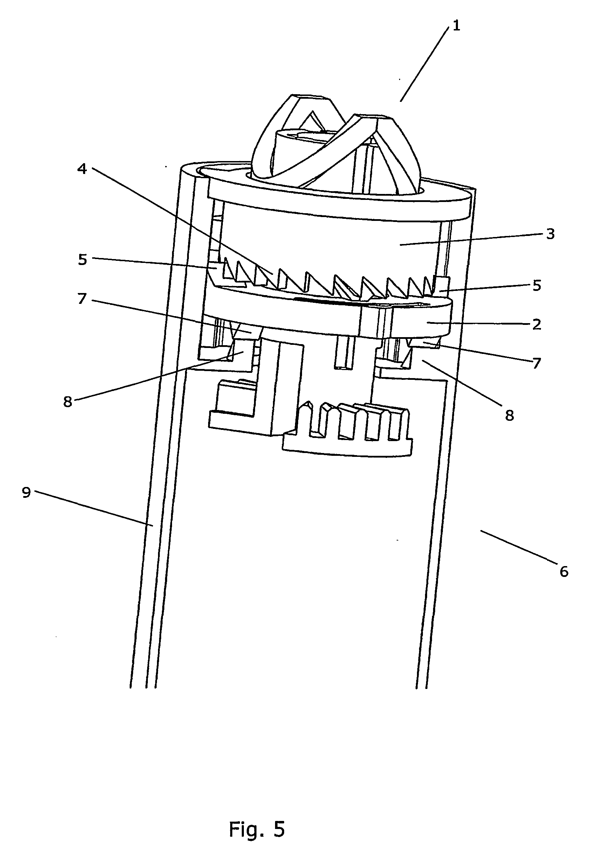

Fig. 5 shows the injection device 6 of Fig. 4. However, in Fig. 5 the tapered members 7, 8 engage. This causes the first part 2 to be moved in a direction towards the second part 3. Thereby the mating teeth 5 are moved into engagement with the set of teeth 4, and the second part 3 is therefore rotated along with the first part 2 until the tapered members 7, 8 are no longer in engagement, whereby the first part 2 will moved in a direction away from the second part 3, etc. Thus, in the injection device 6 shown in Figs. 4 and 5 the second part 3 will be rotated through an angle corresponding to an angle spanned by each of the tapered members 8 on the housing 9 each time the first part 2 is rotated one revolution, i.e. through 360°. When this has occurred a certain number of times, corresponding to the amount of liquid in a cartridge, the stopping surface 14 has been moved to the angular position where it abuts the corresponding surface (not visible) positioned in the housing 9. Thereby the second part 3 will be prevented from further rotation, and a further dose can not be set.

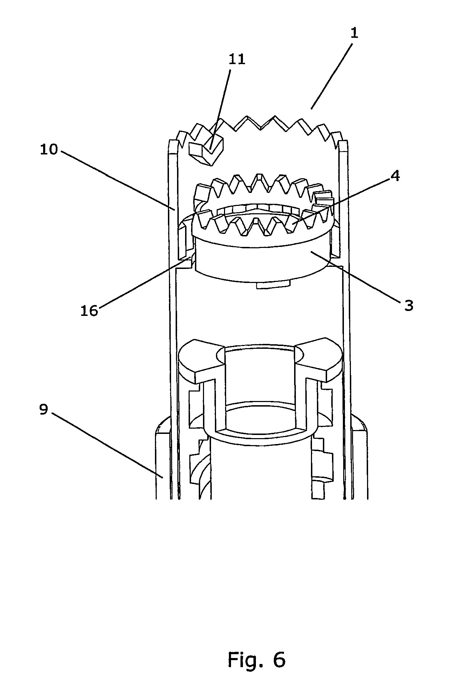

Fig. 6 shows parts of an injection device having a dose limiting mechanism 1 according to a second embodiment of the invention. The dose limiting mechanism 1 comprises a first part (not visible in Fig. 6) and a second part 3. The second part 3 is provided with a set of teeth 4 being adapted to engage with the first part in a manner which will be described further below. The injection device further comprises an outer part 10 which is fixed in relation to a housing 9 during dose setting. The outer part 10 is provided with an activator tap 11 the function of which will be further described below. The second part 3 can rotate relatively to the outer part 10.

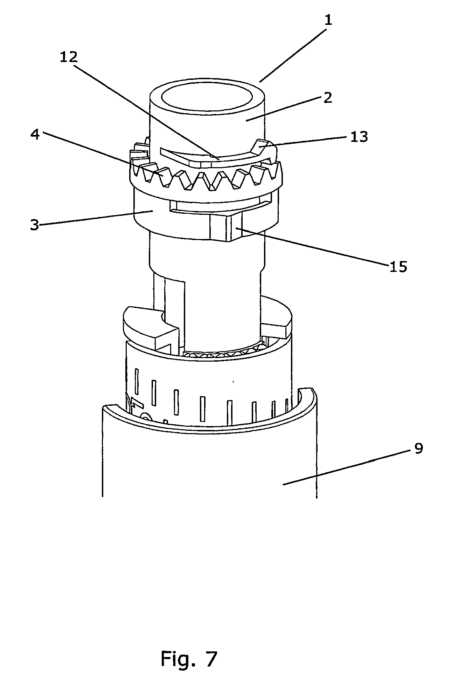

Fig. 7 also shows parts of the injection device of Fig. 6. In the injection device the parts visible in Fig. 7 are positioned inside the parts visible in Fig. 6. It should be noted, however, that the second part 3 is visible in Fig. 6 as well as in Fig. 7, thereby indicating the relative positions of the parts of the two Figures. Fig. 7 shows a first part 2 of the dose limiting mechanism 1. The first part 2 is provided with a flexible arm 12 being adapted to engage with the set of teeth 4 on the second part 3. The first part 2 rotates relatively to the housing 9 and along with a dose setting member (not visible) during dose setting.

The dose limiting mechanism which is shown in Figs. 6 and 7 preferably functions in the following manner. When a dose is being set a dose setting member (not visible) is rotated relatively to the housing 9. The first part 2 is rotated along with the dose setting member. When a protruding part 13 of the flexible arm 12 reaches the position of the activator tap 11, the activator tap 11 will press the flexible arm 12 away from its rest position and towards the second part 3. Thereby the flexible arm 12 will engage the set of teeth 4 on the second part 3, thereby rotating the second part 3 along with it. The rotation of the second part 3 will continue until the protruding part 13 of the flexible arm 12 has passed the position of the activator tap 11. The flexible arm 12 will then move back to its rest position, i.e. out of engagement with the set of teeth 4 of the second part 3. This will cause the rotational movement of the second part 3 to stop. Thus, each time the first part 2 has rotated through one revolution (360°) the second part 3 will be moved through an angle defined by the size of the activator tap 11. In the embodiment shown in Figs. 6 and 7 this angle is 18°.

When this has happened for a specific number of times corresponding to the total amount of liquid contained in a cartridge, a first stop member 15 (Fig. 7) on the second part 3 abuts a stop member (not visible) positioned in a flange 16 (Fig. 6) on the outer part 10. This prevents further rotational movement of the second part 3. This will in turn prevent further rotational movement of the first part 2, and thereby the setting of a further dose. Thereby it is prevented that a dose exceeding the amount of liquid left in the cartridge is set, and the dose limiting mechanism 1 consumes very little space in the injection device.

## Claims

1. A dose setting element for an injection device, the dose setting element being operable to set a desired dose to be injected by the injection device, the dose setting element comprising:
- a first part (2) being adapted to be rotated during the setting of a dose,
**characterised in that** it further comprises:
- a second part (3) being adapted to limit setting of a dose beyond a predefined total amount, the first part (2) and the second part (3) being adapted to interengage in such a way that each time the first part (2) has been rotated through a first specific angle the second part (3) is caused to be rotated through a second specific angle, the second specific angle being smaller than the first specific angle, and
- a stop member (14) being adapted to abut the second part (3) when the second part (3) has been rotated through an angle being equal to the second specific angle a predefined number of times in total, thereby limiting the second part (3) from further rotational movement and thereby preventing further dose setting.

2. A dose setting element according to claim 1, wherein the predefined number of times corresponds to a predefined amount of liquid being left in the cartridge.

3. A dose setting element according to claim 1 or 2, wherein the predefined number of times corresponds to the amount of liquid present in a cartridge.

4. A dose setting element according to any of the preceding claims, further comprising means for moving the first part and/or the second part into a position where the first part and the second part interengage, thereby causing the second part to be rotated along with the first part through an angle being equal to the second specific angle.

5. A dose setting element according to claim 4, wherein the moving means comprises one or more protruding parts positioned on the first part and adapted to cooperate with one or more corresponding protruding parts positioned on the second part, the protruding parts in cooperation causing the movement of the first and/or the second part.

6. A dose setting element according to any of the preceding claims, wherein the first part and the second part are adapted to interengage by means of mating teeth positioned on the first part and the second part, respectively.

7. A dose setting element according to any of claims 1-4, wherein the first part and the second part are adapted to interengage by means of one or more arms positioned on the first or second part and being adapted to engage with one or more corresponding members on the other part.

8. A dose setting element according to claim 7, wherein the one or more arms form(s) part of the first part.

9. A dose setting element according to any of the preceding claims, wherein the first part and the second part are adapted to rotate about a common rotational axis.

10. A dose setting element according to any of the preceding claims, wherein the first part is or forms part of a dose setting member.

11. A dose setting element according to any of the preceding claims, wherein the first part and the second part interengage via at least a third part in such a way that each time the first part has been rotated through a first specific angle the third part is caused to be rotated through a third specific angle, and each time the third part has been rotated through the third specific angle a specific number of times the second part is caused to be rotated through the second specific angle, wherein the third specific angle is smaller than the first specific angle, and wherein the second specific angle is smaller than the third specific angle times the specific number.

12. An injection device comprising:
- a housing,
- a dose setting element according to any of claims 1-11, and
- a piston rod being adapted to cooperate with a piston so as to cause a set dose to be injected by the injection device.

13. An injection device according to claim 12, the injection device having an elongated shape.

## Patentansprüche

1. Dosiseinstellelement für eine Injektionsvorrichtung, wobei das Dosiseinstellelement zum Einstellen einer durch die Injektionsvorrichtung zu injizierenden gewünschten Dosis funktionsfähig ist, wobei das Dosiseinstellelement umfasst:
- ein erstes Teil (2), das derart angepasst ist, während des Einstellens einer Dosis gedreht zu werden,
**dadurch gekennzeichnet, dass** es des Weiteren umfasst:
- ein zweites Teil (3), das derart angepasst ist, das Einstellen einer Dosis über einen vordefinierten Gesamtbetrag hinaus einzuschränken, wobei das erste Teil (2) und das zweite Teil (3) derart angepasst sind, in einer derartigen Weise ineinanderzugreifen, dass jedes Mal, wenn das erste Teil (2) mit einem ersten spezifischen Winkel gedreht worden ist, es bewirkt wird, dass das zweite Teil (3) mit einem zweiten spezifischen Winkel gedreht wird, wobei der zweite spezifische Winkel kleiner als der erste spezifische Winkel ist, und
- ein Stoppelement (14), das derart angepasst ist, dass es an das zweite Teil (3) anstößt, wenn das zweite Teil (3) mit einem Winkel, der gleich dem zweiten spezifischen Winkel ist, eine vordefinierte Gesamtanzahl an Häufigkeiten gedreht worden ist, wodurch das zweite Teil (3) darin eingeschränkt wird, eine weitere Drehung durchzuführen, und es dadurch verhindert wird, dass eine weitere Dosis eingestellt wird.

2. Dosiseinstellelement nach Anspruch 1, wobei die vordefinierte Anzahl an Häufigkeiten einer vordefinierten Menge an in der Patrone zurückgelassener Flüssigkeit entspricht.

3. Dosiseinstellelement nach Anspruch 1 oder 2, wobei die vordefinierte Anzahl an Häufigkeiten der Menge der in der Patrone vorliegender Flüssigkeit entspricht.

4. Dosiseinstellelement nach einem der vorangehenden Ansprüche, des Weiteren umfassend ein Mittel zum Bewegen des ersten Teils und/oder des zweiten Teils in eine Position, in welcher das erste Teil und das zweite Teil ineinandergreifen, wodurch bewirkt wird, dass das zweite Teil zusammen mit dem ersten Teil mit einem Winkel, der gleich dem zweiten spezifischen Winkel ist, gedreht wird.

5. Dosiseinstellelement nach Anspruch 4, wobei das Bewegungsmittel ein oder mehrere hervorstehende Teile umfasst, die an dem ersten Teil positioniert sind und derart angepasst sind, dass sie mit einem oder mehreren entsprechenden hervorstehenden Teilen, die am zweiten Teil positioniert sind zusammenarbeiten, um die Bewegung des ersten und/oder des zweiten Teils zu bewirken.

6. Dosiseinstellelement nach einem der vorangehenden Ansprüche, wobei das erste Teil und das zweite Teil derart angepasst sind, dass sie mittels am ersten Teil bzw. am zweiten Teil positionierter passender Zähne ineinandergreifen.

7. Dosiseinstellelement nach einem der Ansprüche 1-4, wobei das erste Teil und das zweite Teil derart angepasst sind, dass sie mittels eines oder mehrerer Arme ineinandergreifen, der/die am ersten oder zweiten Teil positioniert ist/sind und derart angepasst ist/sind, dass er/sie in ein oder mehrere entsprechende Elemente am anderen Teil eingreift/eingreifen.

8. Dosiseinstellelement nach Anspruch 7, wobei der eine Arm oder die mehreren Arme einen Teil des ersten Teils bildet/bilden.

9. Dosiseinstellelement nach einem der vorangehenden Ansprüche, wobei das erste Teil und das zweite Teil derart angepasst sind, dass sie sich um eine gemeinsame Drehachse drehen.

10. Dosiseinstellelement nach einem der vorangehenden Ansprüche, wobei das erste Teil ein Teil eines Einstellungselements ist oder einen solchen bildet.

11. Dosiseinstellelement nach einem der vorangehenden Ansprüche, wobei das erste Teil und das zweite Teil über mindestens ein drittes Teil in einer derartigen Weise ineinandergreifen, dass jedes Mal, wenn das erste Teil mit einem ersten spezifischen Winkel gedreht worden ist, bewirkt wird, dass das dritte Teil mit einem dritten spezifischen Winkel gedreht wird, und jedes Mal, wenn das dritte Teil mit einem dritten spezifischen Winkel eine spezifische Anzahl an Häufigkeiten gedreht worden ist, bewirkt wird, dass das zweite Teil mit einem zweiten spezifischen Winkel gedreht wird, wobei der dritte spezifische Winkel kleiner als der erste spezifische Winkel ist und wobei der zweite spezifische Winkel kleiner als der dritte spezifische Winkel mal der spezifischen Anzahl ist.

12. Injektionsvorrichtung, umfassend:
- ein Gehäuse,
- ein Dosiseinstellelement nach einem der Ansprüche 1-11 und
- eine Kolbenstange, die derart angepasst ist, dass sie mit einem Kolben derart zusammenarbeitet, dass bewirkt wird, dass eine eingestellte Dosis durch die Injektionsvorrichtung injiziert wird.

13. Injektionsvorrichtung nach Anspruch 12, wobei die Injektionsvorrichtung eine längliche Gestalt aufweist.

## Revendications

1. Un élément de réglage de dose pour un dispositif d'injection, l'élément de réglage de dose pouvant être mis en oeuvre pour régler une dose souhaitée à injecter par le dispositif d'injection, l'élément de réglage de dose comprenant :
- une première pièce (2) qui est apte à être entrainée en rotation durant le réglage d'une dose,
**caractérisé en ce qu'**il comprend en outre :
- une seconde pièce (3) qui est apte à limiter le réglage d'une dose au-delà d'une quantité totale prédéfinie, la première pièce (2) et la seconde pièce (3) étant aptes à venir en engagement mutuel d'une manière telle que chaque fois que la première pièce (2) a été entrainée en rotation d'un premier angle spécifique la seconde pièce (3) soit entrainée en rotation d'un second angle spécifique, le second angle spécifique étant plus faible que le premier angle spécifique, et
- un organe d'arrêt (14) qui est apte à venir en butée contre la seconde pièce (3) lorsque la seconde pièce (3) a été entrainée en rotation d'un angle qui est égal à un nombre total de fois prédéfini le second angle spécifique, limitant ainsi la seconde pièce (3) en un autre mouvement de rotation et empêchant ainsi un réglage de dose supplémentaire.

2. Un élément de réglage de dose selon la revendication 1, dans lequel le nombre de fois prédéfini correspond à une quantité prédéfinie de liquide restant présent dans la cartouche.

3. Un élément de réglage de dose selon la revendication 1 ou 2, dans lequel le nombre de fois prédéfini correspond à la quantité de liquide présent dans une cartouche.

4. Un élément de réglage de dose selon l'une des revendications précédentes, comprenant en outre des moyens pour déplacer la première pièce et/ou la seconde pièce jusqu'à une position où la première pièce et la seconde pièce viennent en engagement mutuel, provoquant ainsi une rotation de la seconde pièce en même temps que la première pièce d'un angle qui est égal au second angle spécifique.

5. Un élément de réglage de dose selon la revendication 4, dans lequel les moyens de déplacement comprennent une ou plusieurs parties saillantes positionnées sur la première pièce et aptes à coopérer avec une ou plusieurs parties saillantes correspondantes positionnées sur la seconde pièce, les parties saillantes en coopération provoquant le mouvement de la première et/ou de la seconde pièce.

6. Un élément de réglage de dose selon l'une des revendications précédentes, dans lequel la première pièce et la seconde pièce sont aptes à venir en engagement mutuel au moyen de dents conjuguées positionnées sur la première pièce et sur la seconde pièce, respectivement.

7. Un élément de réglage de dose selon l'une des revendications 1 à 4, dans lequel la première pièce et la seconde pièce sont aptes à venir en engagement mutuel au moyen d'un ou plusieurs bras positionnés sur la première ou sur la seconde pièce et qui sont aptes à venir s'engager avec un ou plusieurs organes correspondants sur l'autre pièce.

8. Un élément de réglage de dose selon la revendication 7, dans lequel les un ou plusieurs bras font partie de la première pièce.

9. Un élément de réglage de dose selon l'une des revendications précédentes, dans lequel la première pièce et la seconde pièce sont aptes à tourner autour d'un axe de rotation commun.

10. Un élément de réglage de dose selon l'une des revendications précédentes, dans lequel la première pièce est, ou fait partie de, un organe de réglage de dose.

11. Un élément de réglage de dose selon l'une des revendications précédentes, dans lequel la première pièce et la seconde pièce viennent en engagement mutuel via au moins une troisième pièce d'une manière telle que chaque fois que la première pièce a été entrainée en rotation d'un premier angle spécifique la troisième pièce soit entrainée en rotation d'un troisième angle spécifique, et chaque fois que la troisième pièce a été entrainée en rotation du troisième angle spécifique un nombre de fois spécifique la seconde pièce soit entrainée en rotation du second angle spécifique, le troisième angle spécifique étant plus faible que le premier angle spécifique, et le second angle spécifique étant plus faible que le nombre spécifique de fois le troisième angle spécifique.

12. Un dispositif d'injection comprenant :
- un boitier,
- un élément de réglage de dose selon l'une des revendications 1 à 11, et
- une tige de piston apte à coopérer avec un piston de manière à faire en sorte qu'une dose réglée soit injectée par le dispositif d'injection.

13. Un dispositif d'injection selon la revendication 12, où le dispositif d'injection possède une forme allongée.
